# EUROPEAN PATENT SPECIFICATION

(11) **EP 2 288 705 B1**
(45) Date of publication and mention of the grant of the patent: **03.04.2013**
(21) Application number: 08874354.7
(22) Date of filing: 25.11.2008
(51) Int. Cl.: C12N 15/52, C12N 15/82, A01H 1/00, A01H 5/00, A01H 5/10

(54) **USE OF A CHIMERIC GENE 4-CL ENCODING 4-COUMARATE: COA LIGASE IN BRASSICACEAE**
VERWENDUNG EINES FÜR 4-COUMARAT: COA-LIGASE CODIERENDEN CHIMÄREN GENS 4-CL IN BRASSICACEAE
UTILISATION D UN GÈNE CHIMÉRIQUE 4-CL CODANT POUR LA 4-COUMARATE COA LIGASE DANS LES BRASSICACÉES

(30) Priority: 20.05.2008 CN 200810047778
(43) Date of publication of application: 02.03.2011
(73) Proprietor: Oil Crops Research Institute, Chinese Academy Of Agriculture Sciences, Wuhan, Hubei 430062 (CN)
(72) Inventor: WANG, Hanzhong, Wuhan Hubei 430062 (CN); YANG, Xiangdong, Changchun Jilin 130033 (CN); LIU, Guihua, Wuhan Hubei 430062 (CN); WANG, Xinfa, Wuhan Hubei 430062 (CN); HUA, Wei, Wuhan Hubei 430062 (CN); LIU, Jing, Wuhan Hubei 430062 (CN); YANG, Qing, Wuhan Hubei 430062 (CN); ZHENG, Yuanben, Wuhan Hubei 430062 (CN)
(74) Representative: Desomer, Jan G.M.
(86) International application number: PCT/CN2008/073181
(87) International publication number: WO 2009/140839

(56) References cited:
- WO-A1-01/95702
- WO-A1-99/31243
- CN-A- 1 390 940
- CN-A- 1 724 675
- CN-A- 1 733 923
- CN-A- 101 280 315
- US-B1- 6 479 732
- LU HAI ET AL: "Xylem-specific expression of a GRP1.8 promoter::4CL gene construct in transgenic tobacco.", PLANT GROWTH REGULATION, vol. 41, no. 3, November 2003 (2003-11), pages 279-286, XP002633448, ISSN: 0167-6903
- LU X ET AL.: 'Stable and specific expression of 4-coumarate:coenzyme A ligase gene (4CL1) driven by the xylem-specific pto4CLl promoter in the transgenic tobacco' BIOTECHNOL. LETT. vol. 26, 31 July 2004, pages 1147 - 1152, XP008146478
- DATABASE GENBANK [Online] 25 June 2005 YANG X. ET AL.: 'The overexpression of 4-coumarate:CoA ligase in brassica napus boosting their resistance to lodging and sclerotinia sclerotiorm', XP008146480 Database accession no. DQ076679
- DATABASE GENBANK [Online] 14 April 2005 ZHAO H ET AL.: 'Isolation and functional characterization of a cinnamate 4-hydroxylase promoter from populus tomentosa', XP008146479 Database accession no. AY351673
- DATABASE GENBANK [Online] 25 June 2005 YANG X. ET AL.: 'The overexpression of 4-coumarate:CoA ligase in Brassica napus boosting their resistance to lodging and sclerotinia sclerotiorm', XP008146481 Database accession no. AAY84731
- ZHAO H, ET AL.: 'Isolation and functional characterization of a cinnamate 4-hydroxylase promoter from populus tomentosa' PLANT SCI. vol. 168, 17 January 2005, pages 1157 - 1162, XP008146483
- YANG XIANG-DONG ET AL.: 'The cloning, expression and activity assays of 4-CL cDNA the Populus tomentosa' JOURNAL OF HUAZHONG AGRICULTURAL UNIVERSITY vol. 25, no. 2, 30 April 2006, pages 101 - 105, XP008150623
- LEE D. ET AL.: 'Antisense suppression of 4-coumarate:coenzyme A ligase activity in Arabidopsis leads to altered lignin subunit composition' THE PLANT CELL vol. 9, 30 November 1997, pages 1985 - 1998, XP002558237

### SEQUENCE LISTING

<110> Oil Crops Research Institute, Chinese Academy of Agricultural Sciences
   WANG Hanzhong
   YANG Xiangdong
   LIU Guihua
   WANG Xinfa
   HUA Wei
   LIU Jing
   YANG Qing
   ZHENG Yuanben
<120> USE OF A CHIMERIC GENE 4-CL ENCODING 4-COUMARATE:COA LIGASE IN
   BRASSICACEAE
<130> BCS 08-2012
<150> CN200810047778.2 <151> 2008-05-20
<160> 7
<170> PatentIn version 3.3
<210> 1
   <211> 1631
   <212> DNA
   <213> Artificial
<220>
   <223> cDNA nucleotide sequence of the 4-coumarate:CoA ligase from Populus
   tomentosa
<220>
   <221> CDS
   <222> (11)..(1621)
<400> 1
   cgccggatcc atg aat cca caa gaa gaa ttc atc ttt cgc tca aaa tta        49
              Met Asn Pro Gln Glu Glu Phe Ile Phe Arg Ser Lys Leu
              1               5                   10
   cca gac atc tac atc ccg aaa aac ctt ccc ctg cat tca tac gtt ctt       97
   Pro Asp Ile Tyr Ile Pro Lys Asn Leu Pro Leu His Ser Tyr Val Leu
       15                  20                  25
   gaa aac ttg tct aac cat tca tca aaa cct tgc ctg ata aat ggg gcg      145
   Glu Asn Leu Ser Asn His Ser Ser Lys Pro Cys Leu Ile Asn Gly Ala
   30                  35                  40                  45
   aat gga gat gtc tac acc tat gct gac gtt gag ctc aca gca aga aga      193
   Asn Gly Asp Val Tyr Thr Tyr Ala Asp Val Glu Leu Thr Ala Arg Arg
                   50                  55                  60
   gtt gct tct ggt ctt aac aag att ggt att caa caa ggt gac gtg atc      241
   Val Ala Ser Gly Leu Asn Lys Ile Gly Ile Gln Gln Gly Asp Val Ile
               65                  70                  75
   atg ctc ttc cta cca agt tca cct gaa ttc gtg ctt gct ttc cta ggc      289
   Met Leu Phe Leu Pro Ser Ser Pro Glu Phe Val Leu Ala Phe Leu Gly
           80                  85                  90
   gct tca cac aga ggt gcc att atc act gct gcc aat cct ttc tcc acc      337
   Ala Ser His Arg Gly Ala Ile Ile Thr Ala Ala Asn Pro Phe Ser Thr
       95                  100                 105
   cct gca gag cta gca aaa cat gcc aag gcc tcg aga gca aag ctt ctg      385
   Pro Ala Glu Leu Ala Lys His Ala Lys Ala Ser Arg Ala Lys Leu Leu
   110                 115                 120                 125
   ata aca cag gct tgt tac tac gag aag gtt aaa gat ttt gcc cga gaa      433
   Ile Thr Gln Ala Cys Tyr Tyr Glu Lys Val Lys Asp Phe Ala Arg Glu
                   130                 135                 140
   agt gat gtt aag gtc atg tgc gtg gac tct gcc ccg gat ggg tgc ttg      481
   Ser Asp Val Lys Val Met Cys Val Asp Ser Ala Pro Asp Gly Cys Leu
               145                 150                 155
   cac ttt tca gag cta aca cag gca gac gaa aat gaa gcg cct cag gtc      529
   His Phe Ser Glu Leu Thr Gln Ala Asp Glu Asn Glu Ala Pro Gln Val
           160                 165                 170
   gac att agt ccc gat gat gtc gta gca ttg cct tat tca tca ggg act      577
   Asp Ile Ser Pro Asp Asp Val Val Ala Leu Pro Tyr Ser Ser Gly Thr
       175                 180                 185
   aca ggg ttg cca aaa ggg gtc atg tta acg cac aaa ggg cta ata acc      625
   Thr Gly Leu Pro Lys Gly Val Met Leu Thr His Lys Gly Leu Ile Thr
   190                 195                 200                 205
   agt gtt gct caa cag gta gat gga gac aat cct aac ctg tat ttt cac      673
   Ser Val Ala Gln Gln Val Asp Gly Asp Asn Pro Asn Leu Tyr Phe His
                   210                 215                 220
   agt gaa gat gtg att ctg tgt gtg ctg cct atg ttc cat atc tat gct      721
   Ser Glu Asp Val Ile Leu Cys Val Leu Pro Met Phe His Ile Tyr Ala
               225                 230                 235
   ctg aat tca ata atg ctc tgc ggg ctg aga gtc ggt gcc tcg att ttg      769
   Leu Asn Ser Ile Met Leu Cys Gly Leu Arg Val Gly Ala Ser Ile Leu
           240                 245                 250
   ata atg cca aag ttt gag att ggt tct tta ctg gga ttg att gag aag      817
   Ile Met Pro Lys Phe Glu Ile Gly Ser Leu Leu Gly Leu Ile Glu Lys
       255                 260                 265
   tac aag gta tct ata gca ccg gtt gtt cca cct gtg atg atg tca att      865
   Tyr Lys Val Ser Ile Ala Pro Val Val Pro Pro Val Met Met Ser Ile
   270                 275                 280                 285
   gct aag tca cct gat ctt gac aag cat gac ttg tct tct ttg agg atg      913
   Ala Lys Ser Pro Asp Leu Asp Lys His Asp Leu Ser Ser Leu Arg Met
                   290                 295                 300
   ata aaa tct gga ggg gct cca ttg ggc aag gaa ctt gaa gat act gtc      961
   Ile Lys Ser Gly Gly Ala Pro Leu Gly Lys Glu Leu Glu Asp Thr Val
               305                 310                 315
   aga gct aag ttt cct cag gct aga ctt ggt cag gga tat gga atg acc     1009
   Arg Ala Lys Phe Pro Gln Ala Arg Leu Gly Gln Gly Tyr Gly Met Thr
           320                 325                 330
   gag gca gga cct gtt cta gca atg tgc ttg gca ttt gcc aag gaa cca      1057
   Glu Ala Gly Pro Val Leu Ala Met Cys Leu Ala Phe Ala Lys Glu Pro
       335                 340                 345
   ttc gac ata aaa cca ggt gca tgt ggg act gta gtc agg aat gca gag      1105
   Phe Asp Ile Lys Pro Gly Ala Cys Gly Thr Val Val Arg Asn Ala Glu
   350                 355                 360                 365
   atg aag att gtt gac cca gaa aca ggg gcc tct cta ccg agg aac cag      1153
   Met Lys Ile Val Asp Pro Glu Thr Gly Ala Ser Leu Pro Arg Asn Gln
                   370                 375                 380
   cct ggt gag atc tgc atc cgg ggt gat cag atc atg aaa gga tat ctt      1201
   Pro Gly Glu Ile Cys Ile Arg Gly Asp Gln Ile Met Lys Gly Tyr Leu
               385                 390                 395
   aat gac cct gag gca acc tca aga aca ata gac aaa gaa gga tgg ctg      1249
   Asn Asp Pro Glu Ala Thr Ser Arg Thr Ile Asp Lys Glu Gly Trp Leu
           400                 405                 410
   cac aca ggc gat atc ggc tac att gat gat gat gat gag ctt ttc atc      1297
   His Thr Gly Asp Ile Gly Tyr Ile Asp Asp Asp Asp Glu Leu Phe Ile
       415                 420                 425
   gtt gac aga ttg aag gaa ttg atc aag tat aaa ggg ttt cag gtt gct      1345
   Val Asp Arg Leu Lys Glu Leu Ile Lys Tyr Lys Gly Phe Gln Val Ala
   430                 435                 440                 445
   cct gct gaa ctc gaa gct ctg tta ata gcc cat cca gag ata tcc gat      1393
   Pro Ala Glu Leu Glu Ala Leu Leu Ile Ala His Pro Glu Ile Ser Asp
                   450                 455                 460
   gct gct gta gta gga ttg aaa gat gag aat gcg gga gaa gtt cct gtt      1441
   Ala Ala Val Val Gly Leu Lys Asp Glu Asn Ala Gly Glu Val Pro Val
               465                 470                 475
   gca ttt gta gtg aaa tca gaa aag tct cag gcc acc gaa gat gaa att      1489
   Ala Phe Val Val Lys Ser Glu Lys Ser Gln Ala Thr Glu Asp Glu Ile
           480                 485                 490
   aag cag tat att tca aaa cag gtg ata ttc tac aag aga ata aaa cga      1537
   Lys Gln Tyr Ile Ser Lys Gln Val Ile Phe Tyr Lys Arg Ile Lys Arg
       495                 500                 505
   gtt ttc ttc att gaa gct att ccc aag gca cca tct ggc aag atc ctg      1585
   Val Phe Phe Ile Glu Ala Ile Pro Lys Ala Pro Ser Gly Lys Ile Leu
   510                 515                 520                 525
   agg aag aat ctg aaa gaa aag ttg gcg ggc ata taa gagctcctcg           1631
   Arg Lys Asn Leu Lys Glu Lys Leu Ala Gly Ile
                   530                 535
<210> 2
   <211> 536
   <212> PRT
   <213> Artificial
<220>
   <223> Synthetic Construct
<400> 2
   Met Asn Pro Gln Glu Glu Phe Ile Phe Arg Ser Lys Leu Pro Asp Ile
   1               5                   10                  15
   Tyr Ile Pro Lys Asn Leu Pro Leu His Ser Tyr Val Leu Glu Asn Leu
               20                  25                  30
   Ser Asn His Ser Ser Lys Pro Cys Leu Ile Asn Gly Ala Asn Gly Asp
           35                  40                  45
   Val Tyr Thr Tyr Ala Asp Val Glu Leu Thr Ala Arg Arg Val Ala Ser
       50                  55                  60
   Gly Leu Asn Lys Ile Gly Ile Gln Gln Gly Asp Val Ile Met Leu Phe
   65                  70                  75                  80
   Leu Pro Ser Ser Pro Glu Phe Val Leu Ala Phe Leu Gly Ala Ser His
                   85                  90                  95
   Arg Gly Ala Ile Ile Thr Ala Ala Asn Pro Phe Ser Thr Pro Ala Glu
               100                 105                 110
   Leu Ala Lys His Ala Lys Ala Ser Arg Ala Lys Leu Leu Ile Thr Gln
           115                 120                 125
   Ala Cys Tyr Tyr Glu Lys Val Lys Asp Phe Ala Arg Glu Ser Asp Val
       130                 135                 140
   Lys Val Met Cys Val Asp Ser Ala Pro Asp Gly Cys Leu His Phe Ser
   145                 150                 155                 160
   Glu Leu Thr Gln Ala Asp Glu Asn Glu Ala Pro Gln Val Asp Ile Ser
                   165                 170                 175
   Pro Asp Asp Val Val Ala Leu Pro Tyr Ser Ser Gly Thr Thr Gly Leu
               180                 185                 190
   Pro Lys Gly Val Met Leu Thr His Lys Gly Leu Ile Thr Ser Val Ala
           195                 200                 205
   Gln Gln Val Asp Gly Asp Asn Pro Asn Leu Tyr Phe His Ser Glu Asp
       210                 215                 220
   Val Ile Leu Cys Val Leu Pro Met Phe His Ile Tyr Ala Leu Asn Ser
   225                 230                 235                 240
   Ile Met Leu Cys Gly Leu Arg Val Gly Ala Ser Ile Leu Ile Met Pro
                   245                 250                 255
   Lys Phe Glu Ile Gly Ser Leu Leu Gly Leu Ile Glu Lys Tyr Lys Val
               260                 265                 270
   Ser Ile Ala Pro Val Val Pro Pro Val Met Met Ser Ile Ala Lys Ser
           275                 280                 285
   Pro Asp Leu Asp Lys His Asp Leu Ser Ser Leu Arg Met Ile Lys Ser
       290                 295                 300
   Gly Gly Ala Pro Leu Gly Lys Glu Leu Glu Asp Thr Val Arg Ala Lys
   305                 310                 315                 320
   Phe Pro Gln Ala Arg Leu Gly Gln Gly Tyr Gly Met Thr Glu Ala Gly
                   325                 330                 335
   Pro Val Leu Ala Met Cys Leu Ala Phe Ala Lys Glu Pro Phe Asp Ile
               340                 345                 350
   Lys Pro Gly Ala Cys Gly Thr Val Val Arg Asn Ala Glu Met Lys Ile
           355                 360                 365
   Val Asp Pro Glu Thr Gly Ala Ser Leu Pro Arg Asn Gln Pro Gly Glu
       370                 375                 380
   Ile Cys Ile Arg Gly Asp Gln Ile Met Lys Gly Tyr Leu Asn Asp Pro
   385                 390                 395                 400
   Glu Ala Thr Ser Arg Thr Ile Asp Lys Glu Gly Trp Leu His Thr Gly
                   405                 410                 415
   Asp Ile Gly Tyr Ile Asp Asp Asp Asp Glu Leu Phe Ile Val Asp Arg
               420                 425                 430
   Leu Lys Glu Leu Ile Lys Tyr Lys Gly Phe Gln Val Ala Pro Ala Glu
           435                 440                 445
   Leu Glu Ala Leu Leu Ile Ala His Pro Glu Ile Ser Asp Ala Ala Val
       450                 455                 460
   Val Gly Leu Lys Asp Glu Asn Ala Gly Glu Val Pro Val Ala Phe Val
   465                 470                 475                 480
   Val Lys Ser Glu Lys Ser Gln Ala Thr Glu Asp Glu Ile Lys Gln Tyr
                   485                 490                 495
   Ile Ser Lys Gln Val Ile Phe Tyr Lys Arg Ile Lys Arg Val Phe Phe
               500                 505                 510
   Ile Glu Ala Ile Pro Lys Ala Pro Ser Gly Lys Ile Leu Arg Lys Asn
           515                 520                 525
   Leu Lys Glu Lys Leu Ala Gly Ile
       530                 535
<210> 3
   <211> 1117
   <212> DNA
   <213> Artificial
<220>
   <223> promoter region of the cinnamate-4-hydroxylase gene from Populus tomentosa
<400> 3
   ctctgcagcc aagttagcca gacatgaaaa ggtgagaaaa acattttttg acataagaaa      60
   ctaacgttgc ttggatttaa gatagaatta ttgaccttgt aagaagacaa acgtattctt    120
   ggaaattaac atgtattaat aaatgcaaag tagtttgtca cactatggga taaaatctct    180
   aataaaaagt aagaccttat agtttcaaga ggttaggttg atatttaaag agagatttct    240
   tttataactt tttaggttga aatcttgaaa ttaatattaa aaagatttgt taatcatttt    300
   cttttaaata ctttggattg atgccagggg tttacattta aaattattct taaatgaaac    360
   ttgatggtgt gtgtttgata ttgtattttc acatgttttt aaaatgaatt tgtttttaaa    420
   aaatatcaaa ttaataattt ttttattttt tttcaaagat tttaatgtat taattttaaa    480
   aataaaataa aaattatttt aatatatttt taaataaaaa atattttcaa ataaaaaaat    540
   taaaaaataa aaagatagga aaaaaacttt cttggttgag ggcctcattc cttgaaattt    600
   aagaaagcgt ttgggaactc ggtgcaaacc gtgttcctat taaatttgaa tttttttttg     660
   ttaaaattta atgcggttta tattttttgg atcgttttga tgtgctgata tcaaaaataa    720
   ttttaaaaaa ataaaaaaaa ttattaatat gtatttcaac acgaaaagtt atttgaaaaa    780
   caatcaccac cccattgatt cacacgttct aaattatcat aaattagtgg cgcccacatt    840
   acatcttgaa tagaaataca aaaaggccag gcaaattaat taatatggtg accatatgac    900
   attttcagcc accaacccgc cttacctact actatccatg attatcaata acagtctcct    960
   accacctcaa atgtaacgcc gttaactctc tctctctccc ccacacacac aacccaacgc   1020
   gtgaaattca acttcatttc ctctctaatt tttgcagctt ataaaaccca agctctcctc   1080
   atcctgttgc tcccatccaa ccatcactct ctagaac                            1117
<210> 4
   <211> 24
   <212> DNA
   <213> Artificial
<220>
   <223> Forward primer for the PCR amplication of the 4CL gene of Populus
   tomentosa
<400> 4
   atgaatccac aagaagaatt catc 24
<210> 5
   <211> 27
   <212> DNA
   <213> Artificial
<220>
   <223> Reverse primer for the PCR amplication of the 4CL gene of Populus
   tomentosa
<400> 5
   ttatatgcct gccaactttt ctttcag 27
<210> 6
   <211> 27
   <212> DNA
   <213> Artificial
<220>
   <223> forward primer for detection of the chimeric C4H-4CL gene
<400> 6
   cagcttcttc tgctttcaat tactctc 27
<210> 7
   <211> 22
   <212> DNA
   <213> Artificial
<220>
   <223> reverse primer for detection of the chimeric gene C4H-4CL
<400> 7
   caacaggaac ttctcccgca tt 22

## Description

### Field of the Invention

The present invention relates to the field of molecular biology. In particular, it relates to the use of a chimeric gene encoding 4-coumarate CoA ligase ("4-CL") in Brassicaceae plants, such as *Brassica napus.*

### Background of the Invention

Lignin, as a complex phenylpropane polymer in plant, is one of the main components of mechanical tissue and cell wall of plant, and it plays an important role in aspects of plant growth and development and resistance to adversity. In the process of cell wall lignification, lignin permeates into cell wall, and fills up the cell wall skeleton, thus enhances the hardness of cell wall, and strengthens the mechanical support and compressive strength of the cell. In the interaction of host with pathogen, cell wall lignification is one of the characteristics of anti-disease response. Lignin, as an important physical anti-pathogen material in plant, can deposit on hydroxyproline-rich glycoprotein (HRGP) in cell wall, to strengthen cell wall and protect cell from pathogen infection as a structural barrier.

For the past few years, study on the regulation of lignin biosynthesis with mutants or transgenic plants mainly takes place at three levels: the first is the regulation of phenylalanine pathway, which involves 3 enzymes: PAL (phenylalanine ammonia-lyase), C4H (cinnamate 4-hydroxylase) and 4-CL (4-coumarate: CoA ligase), their expression determines the total amount of lignin; the second is the regulation of specific synthesis pathway of lignin, which mainly focuses on 3 enzymes, i.e. COMT (caffeic acid O-methyltransferase), CCoAOMT (caffeoyl-CoA O-methyltransferase) and F5H (ferulate 5-hydroxylase), expression of these enzymes has impact on lignin content and especially on specific synthesis of lignin monomer, and determines the ratio of each monomer in total lignin; the third is the regulation of enzymes downstream of the specific synthesis pathway of lignin, which includes two reductases: CAD (cinnamyl alcohol dehydrogenase) and CCR (cinnamoyl-CoA reductase), they are responsible for reducing various hydroxycinnamoyl-coenzyme A (CoA) esters to respective lignin monomers.

Phenylalanine pathway is one of the three secondary metabolism pathways of plant. A lot of secondary metabolism products of plant come from this pathway including lignin, flavanoid, anthocyanidin, phytoalexin, phenols, and signaling molecules. This pathway consists of three enzymes, PAL, C4H and 4-CL, wherein 4-CL is responsible for catalyzing cinnamic acids and their derivatives to produce respective coenzyme A esters, the thus activated substrates then enter various metabolic pathways, and take part in synthesis of various downstream secondary products.

Since 4-CL is at the metabolic branching point in the phenylalanine pathway, and is the key enzyme in the lignin monomer synthesis pathway, it is considered the ideal target gene for regulating lignin synthesis. At present, studies on 4-CL regulation are mostly aimed at reducing the activity level of endogenous 4-CL of plant by antisense RNA technique, so as to reduce lignin content, and improve paper-making quality of the wood, and feeding value of the pasture.

Lu et al. (2004, Biotechnology Letters 26, 14 pp 1147-1152) described the introduction of a gene encoding the 4-CL from *Populus tomentosa* under control of its own promoter in tobacco. The transgenic tobacco lines had an increased lignin content in the stem (25%) but there was no increase of lignin content in the leaves.

Research on aspects of increasing lignin content in Brassicaceae plants, and improving resistance to adverse environment by 4-CL overexpression has never been reported.

There are many genes or cDNAs encoding 4-CL protein isolated from various plants publicly available,. The nucleotide sequence of the cDNA of Populus tomentosa and the amino acid sequence of the protein have been made publicly available under accession number DQ076679 and AAY84731.

There remains a need in the art to increase the resistance of Brassicaceae plants, particularly Brassica plants against fungal diseases, as well as to increase the resistance of such plants to lodging.

### Summary of the Invention

An object of this invention is to provide a chimeric gene comprising a coding region of the lignin monomer synthetic gene *4-CL* from *Populus tomentosa* under control of a wound-inducible promoter, such as a wound-inducible and xylem-selective promoter such as the promoter of the cinnamate-4-hydroxylase gene. The 4-CL gene is the key gene for regulating the total amount of lignin synthesized, responsible for catalyzing various cinnamic acids and their derivatives to produce respective cinnamic coenzyme A esters, and involved in synthesis of various downstream secondary products, thus increases the total amount of lignin, and improves the ability of plant to resist pathogenic infection.

Another object of this invention is to provide the use of a chimeric gene encoding *4-CL* such as the *4-CL* gene from *Populus tomentosa,* in resistance of Brassicaceaea plants, such as *Brassica napus,* against fungal disease caused by *Sclerotinia sclerotiorum.*

The invention also provides plants and plant cells, preferably Brassica plant cells and plants, comprising a chimeric gene comprising a DNA fragment encoding 4-CL under control of a wound-inducible plant-expressible promoter and methods to produce such Brassica plants.

### Brief Description of the Drawings

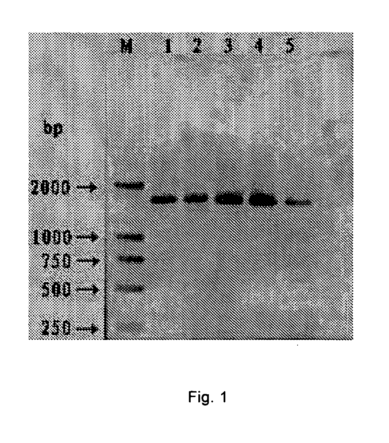

Fig.1 Picture of gel-electrophoresis of *4-CL* cDNA target fragments amplified by RT-PCR.
Fig.2 Schematic diagram of construction of the plant expression vector. Kan: kanamycin resistance, C4H: promoter fragment, *4-CL:* cDNA clone of the Populus tomentosa 4-coumarate:CoA ligage is inserted downstream of the C4H promotor.
Fig.3: Panel A: Rooted shoots of a transgenic *B. napus* comprising the C4H-4CL chimeric gene; Panel B: Transgenic *B. napus* plants transferred to soil containers.
Fig.4 PCR results of *4-CL* transgenic *Brassica napus* with C4H:4CL chimeric gene. Lanes 5-27 are samples from tranformed plants, - is wild plant control, + is positive control. The result shows that the chimeric 4-CL gene has been successfully introcuded in the majority of the transformed plant lines.
Fig.5 Comparison of transgenic B. napus plants and control plants after inoculation with *Sclerotinia sclerotiorum.*
Fig.6 Schematic representation of the stem strength of transgenic B. napus plant lines and control plants.

### Detailed Description of Various Embodiments of the Invention

The invention is based on the observation by the inventors that transgenic Brassica plants comprising a chimeric gene comprising a DNA fragment encoding 4-CL under control of a heterologous plant-expressible promoter (such as a plant expressible promoter driving xylem-specific transcription and/or a wound-inducible plant-expressible promoter) had an increased content of lignin, and furthermore exhibited increased resistance to infection by the fungal pathogen Slerotinia sclerotiorum as well as increased resistance to lodging.

In a first embodiment, the invention thus provides a method to increase resistance against fungal disease caused by Sclerotinia sclerotiorum in a Brassica plant comprising the step of providing cells of said Brassica plant with a chimeric gene, said chimeric gene comprising a nucleotide sequence encoding a 4-coumarate-CoA ligase, preferably a 4-coumarate-CoA ligase from plant origin, operably linked to a heterologous plant-expressible promoter and a transcription termination and polyadenylation region, wherein said plant-expressible promoter is a wound-inducible plant-expressible promoter, such as a wound-inducible and xylem selective plant-expressible promoter.

As used herein "4-coumarate-CoA ligase" refers to an enzyme (EC 6.2.1.12) that catalyzes the chemical reaction:

ATP + 4-coumarate + CoA ⇄ AMP + diphosphate + 4-coumaroyl-CoA

This enzyme belongs to the family of ligases, specifically those forming carbon-sulfur bonds as acid-thiol ligases. The systematic name of this enzyme class is 4-coumarate:CoA ligase (AMP-forming). Other names in common use include 4-coumaroyl-CoA synthetase, p-coumaroyl CoA ligase, p-coumaryl coenzyme A synthetase, p-coumaryl-CoA synthetase, p-coumaryl-CoA ligase, feruloyl CoA ligase, hydroxycinnamoyl CoA synthetase, 4-coumarate:coenzyme A ligase, caffeolyl coenzyme A synthetase, p-hydroxycinnamoyl coenzyme A synthetase, feruloyl coenzyme A synthetase, sinapoyl coenzyme A synthetase, 4-coumaryl-CoA synthetase, hydroxycinnamate:CoA ligase, p-coumaryl-CoA ligase, p-hydroxycinnamic acid:CoA ligase, and 4CL. This enzyme participates in phenylpropanoid biosynthesis.

In one embodiment, a DNA fragment encoding 4CL from Populus tomentosa is used, such as a DNA fragment comprising the nucleotide sequence of SEQ ID NO. 1 from nucleotide 11 to nucleotide 1621. However, having read the current invention, the person skilled in the art will realize that other DNA fragments encoding other 4CL enzymes (from plant origin) can be used to similar effect.

Alternative 4-CL encoding nucleic acids suitable for the methods and means of the current invention are nucleotide sequences encoding 4-CL proteins having an amino acid sequence which may be found in amino acid sequence databases such as the entries with the following identification numbers: AAB42382 *(Pinus taeda*); AAB42383(*Pinus taeda*); AAC24503 *(Populus tremuloides);* AAC24504 *(Populus tremuloides);* AAF37732 *(Lolium perenne);* AAF37733 *(Lolium perenne);* AAF37734 *(Lolium perenne);* AAG46175 (Oryza *sativa*); AAG50881 *(Arabidopsis* tha*l*iana); AAL24191 *(Arabidopsis thaliana*); AAL30738 *(Oryza sativa*); AAL35216 *(Amorpha fruticosa*); AAL90967 *(Arabidopsis thaliana*); AAL91633 *(Arabidopsis thaliana*); AAM19949 *(Arabidopsis thaliana*); AAM91412 *(Arabidopsis thaliana*); AAN18181 *(Arabidopsis thaliana*); AAO64847 *(Arabidopsis thaliana*); AAQ56837 *(Arabidopsis thaliana*); AAS82605 *(Zea mays*); AAT02218 *(Agastache rugosa*); AAW66826 *(Zingiber officinale*); ABB90402 *(Leucaena* leucocephala); ABI26719 *(Hibiscus cannabinus);* ABJ98946 *(Arabidopsis thaliana);* ABL01799 *(Leucaena leucocephala);* ABY89349 *(Pinus taeda);* ABY89350 *(Pinus taeda);* ABY89351 *(Pinus taeda);* ABY89352 *(Pinus taeda);* ABY89353 *(Pinus taeda);* ABY89354 *(Pinus taeda);* ABY89355 *(Pinus taeda);* ABY89356 *(Pinus taeda);* ABY89357 *(Pinus taeda);* ABY89358 *(Pinus taeda);* ABY89359 *(Pinus taeda);* ABY89360 *(Pinus taeda);* ABY89361 *(Pinus taeda);* ABY89362 *(Pinus taeda);* ABY89363 *(Pinus taeda);* ABY89364 *(Pinus taeda);* ABY89365 *(Pinus taeda);* ABY89366 *(Pinus taeda);* ABY89367 *(Pinus taeda);* ABY89368 *(Pinus taeda);* ABY89369 *(Pinus taeda);* ABY89370 *(Pinus taeda);* ABY89371 *(Pinus taeda);* ABY89372 *(Pinus taeda);* ABY89373 *(Pinus taeda);* ABY89374 *(Pinus taeda);* ABY89375 *(Pinus taeda);* ABY89376 *(Pinus taeda);* ABY89377 *(Pinus taeda);* ABY89378 *(Pinus taeda);* ABY89379 *(Pinus taeda);* ABY89380 *(Pinus taeda);* ABY89381 *(Pinus radiata);* ABY89382 *(Pinus radiata);* ABY89383 *(Pinus sylvestris);* ABY89384 *(Pinus sylvestris);* ABY89385 *(Pinus elliottii);* ABY89386 *(Pinus elliottii);* CAK22404 *(Picea abies);* P0C5B6 *(Arabidopsis thaliana);* P17814 *(Oryza sativa);* P41636 *(Pinus taeda);* Q0DV32 *(Oryza sativa);* Q10S72 *(Oryza sativa);* Q336M7 *(Oryza sativa);* Q3E6Y4 *(Arabidopsis thaliana);* Q42524 *(Arabidopsis thaliana);* Q42982 *(Oryza sativa);* Q67W82 *(Oryza sativa);* Q69RG7 *(Oryza sativa);* Q6ETN3 *(Oryza sativa);* Q6YYZ2 *(Oryza sativa);* Q6ZAC1 *(Oryza sativa);* Q7F1X5 *(Oryza sativa);* Q7XXL2 *(Oryza sativa);* Q84P21 *(Arabidopsis thaliana);* Q84P23 *(Arabidopsis thaliana);* Q84P24 *(Arabidopsis thaliana);* Q84P25 *(Arabidopsis thaliana);* Q84P26 *(Arabidopsis thaliana);* Q8GVF9 *(Oryza sativa);* Q8RU95 *(Oryza sativa);* Q9LQ12 *(Arabidopsis thaliana);* Q9LU36 *(Arabidopsis thaliana);* Q9M0X9 *(Arabidopsis thaliana);* Q9S725*(Arabidopsis thaliana);* Q9S777*(Arabidopsis thaliana);* AAL56850 *(Populus tomentosa);* AAL02145 *(Populus tomentosa);* AAL02144 *(Populus tomentosa);* AAY84731 *(Populus tomentosa);* AAS88873 *(Populus tomentosa).*

Examples of nucleotide sequences encoding 4-CL proteins, suitable for the methods of the current invention can be found in nucleotide sequence databases as the entries with the following identification numbers: EU224389 *(Ruta graveolens);* EU224388 *(Ruta graveolens);* AL161502 *(Arabidopsis thaliana);* NC_003070 *(Arabidopsis thaliana);* NC_003076 *(Arabidopsis thaliana);* NC_003075 *(Arabidopsis thaliana);* NC_003074 *(Arabidopsis thaliana);* NC_003071 *(Arabidopsis thaliana* chromosome); EU196764 *(Ruta graveolens);* EU196763 *(Ruta graveolens);* BV728707 *(Pinus taeda);* X05353 *(Petroselinum crispum);* X13325 *(Petroselinum crispum);* DQ093070 (*Populus deltoides);* NM_101901 *(Arabidopsis thaliana);* NM_179513 *(Arabidopsis thaliana);* NM_123172 *(Arabidopsis thaliana);* NM_113019 *(Arabidopsis thaliana);* NM_105180 *(Arabidopsis thaliana);* NM_104972 *(Arabidopsis thaliana);* NM_101898 *(Arabidopsis thaliana);* NM_001084228 *(Arabidopsis thaliana);* NM_202143 *(Arabidopsis thaliana);* NM_116755 *(Arabidopsis thaliana);* NM_113018 *(Arabidopsis thaliana);* NM_101899 *(Arabidopsis thaliana);* NM_125733 *(Arabidopsis thaliana);* NM_179462 *(Arabidopsis thaliana);* NM_104046 *(Arabidopsis thaliana);* NM_101900 *(Arabidopsis thaliana);* NM_113051 *(Arabidopsis thaliana);* NM_118019 *(Arabidopsis thaliana);* BV728981(*Pinus taeda);* EU603323 *(Populus trichocarpa);* NC_008401 *(Oryza sativa* (japonica cultivar-group)); NM_001068470 *(Oryza sativa* (japonica cultivar-group) ); NM_001067888 *(Oryza sativa* (japonica cultivar-group)); NC_008399 *(Oryza sativa* (japonica cultivar-group)); NM_001064787 *(Oryza sativa* (japonica cultivar-group)); NC_008397 *(Oryza sativa* (japonica cultivar-group)); NM_001060839 *(Oryza sativa* (japonica cultivar-group)); NM_001058971 *(Oryza sativa* (japonica cultivar-group)); NC_008396 *(Oryza sativa* (japonica cultivar-group)); NM_001055527 *(Oryza sativa* (japonica cultivar-group)); NM_001055402 *(Oryza sativa* (japonica cultivar-group)); NC_008395 *(Oryza sativa* (japonica cultivar-group)); NM_001054354 *(Oryza sativa* (japonica cultivar-group)); NM_001052604 *(Oryza sativa* (japonica cultivar-group)); NC_008394 *(Oryza sativa* (japonica cultivar-group)); NM_001051646 *(Oryza sativa* (japonica cultivar-group)); AB234050 *(Oryza sativa);* NM_001111788 (Zea mays); EF589315 *(Oryza sativa* (indica cultivar-group)); EF589314 *(Oryza sativa* (indica cultivar-group)); EF589313 *(Oryza sativa* (indica cultivar-group)); EF589285 *(Oryza sativa* (indica cultivar-group)); EF589284 *(Oryza sativa* (indica cultivar-group)); DQ400697 *(Amebia euchroma);* AP008214 *(Oryza sativa* (japonica cultivar-group)); AP008212 *(Oryza sativa* (japonica cultivar-group)); AP008210 *(Oryza sativa* (japonica cultivar-group)); AP008209 *(Oryza sativa* (japonica cultivar-group)); AP008208 *(Oryza sativa* (japonica cultivar-group)); AP008207 *(Oryza sativa);* AY237164 *(Salvia miltiorrhiza);* AL161549 *(Arabidopsis thaliana);* AL132967 *(Arabidopsis thaliana);* AL021711 *(Arabidopsis thaliana);* X52623 (Oryza *sativa);* X13324 *(Petroselinum crispum);* X69954 *(Glycine max* L.); AF466202 *(Zea mays);* AC079736 *(Oryza sativa);* DQ147001 *(Eucalyptus camaldulensis);* DQ076679(*Populus tomentosa);* X05351 *(Petroselinum crispum);* X05350 *(Petroselinum crispum);* AB166768 *(Scutellaria baicalensis);* AB166767(*Scutellaria baicalensis);* AC079830 (Oryza *sativa);* AY566301 *(Zea mays);* AB007649 *(Arabidopsis thaliana);* AC073867 *(Oryza sativa);* AC079887 *(Oryza sativa);* AC078840 *(Oryza sativa);* AC022457 *(Oryza sativa);* AC025783 (Oryza *sativa);* AC068924 *(Oryza sativa);* AC084320 *(Oryza sativa);* AC091247 *(Oryza sativa);* AC091123 *(Oryza sativa);* AC087192 *(Oryza sativa);* AC024594 *(Oryza sativa);* AC084406 *(Oryza sativa);* AC025294 *(Arabidopsis thaliana);* AC018727 *(Oryza sativa);* AC026815 *(Oryza sativa);* AC069145 *(Oryza sativa);* AC051634 *(Oryza sativa);* AC027665 *(Arabidopsis thaliana);* BX784388 *(Pinus pinaster);* AY376735 *(Arabidopsis thaliana);* AY376734 *(Arabidopsis thaliana);* AY376733 *(Arabidopsis thaliana);* AY376732 *(Arabidopsis thaliana);* AY376731 *(Arabidopsis thaliana);* AY376730 *(Arabidopsis thaliana);* AY376729 *(Arabidopsis thaliana);* AY376728 *(Arabidopsis thaliana);* BT010394 *(Arabidopsis thaliana);* AY163489 *(Nicotiana sylvestris);* BT005912 *(Arabidopsis thaliana);* AY163490 *(Nicotiana sylvestris);* AF002257 *(Glycine max);* AF150686 *(Solanum tuberosum);* BT000614 *(Arabidopsis thaliana);* AY133582 *(Arabidopsis* tha*l*iana); AY095992 *(Arabidopsis thaliana);* AF279267 *(Glycine max);* AY075622 *(Arabidopsis thaliana);* AF002259 *(Glycine max);* AY090306 *(Arabidopsis thaliana);* AF314180 *(Populus tomentosa);* AF435968 *(Amorpha fruticosa);* AY058083 *(Arabidopsis thaliana);* AY043495 *(Populus tomentosa); AY043494 (Populus tomentosa);* AF283552 *(Populus balsamifera* subsp. *trichocarpa x Populus deltoides);* AF008184 *(Populus x generosa);* AF144503 *(Pinus armandii);* AF144502 *(Pinus armandii);* AF144501 *(Pinus armandii);* D49367 (Lithospermum *erythrorhizon);* AF239687 *(Rubus idaeus);* AF239686 *(Rubus idaeus);* AF239685 *(Rubus idaeus);* AF052223 *(Lolium perenne);* AF052222 *(Lolium perenne);* AF052221 *(Lolium perenne);* U18675 *(Arabidopsis thaliana);* AF106088 *(Arabidopsis thaliana);* AF106087 *(Arabidopsis thaliana);* AF106085 *(Arabidopsis thaliana);* AF106084 *(Arabidopsis thaliana);* AF041051 *(Populus tremuloides);* AF041050 *(Populus tremuloides);* AF041049 *(Populus tremuloides);* D49366 *(Lithospermum erythrorhizon);* X05352 *(Petroselinum crispum);* L43362 *(Oryza sativa);* M62755 *(Solanum tuberosum).*

Nucleotide sequences of partial cDNA clones encoding 4-CL proteins (which can be used to isolate full length cDNA clones, the latter suitable for use according to the current invention) can also be found in nucleotide sequence databases as the entries with the following identification numbers: AF207575 *(Brassica oleracea);* AF207574 *(Brassica rapa);* AF207573 *(Brassica rapa);* AF207572 *(Brassica oleracea);* AF207571 *(Brassica rapa);* AF207570 *(Brassica rapa);* AF207569 *(Brassica napus);* AF207568 *(Brassica napus);* AF207567 *(Brassica napus);* AF207566 *(Brassica napus);* AF207565 *(Brassica napus);* AF207564 *(Brassica napus);* EU183423 *(Thujopsis dolabrata);* EU183422 *(Thuja occidentalis);* EU 183421 *(Thuja sutchuenensis) ;* EU183420 *(Thuja standishii);* EU183419 *(Thuja koraiensis);* EU183418 *(Thuja plicata);* EU183417 *(Thuja plicata);* AF150687 *(Solanum tuberosum);* AF270934 *(Rubus idaeus);* AF270933 *(Rubus idaeus);* AF144529 *(Cedrus atlantica);* AF144528 *(Pseudolarix amabilis);* AF144527 *(Pseudolarix amabilis);* AF144526 *(Tsuga canadensis),;* AF144525 *(Tsuga canadensis);* AF144524 *(Tsuga mertensiana);* AF144523 *(Nothotsuga longibracteata);* AF144522 *(Keteleeria evelyniana);* AF144521 *(Abies beshanzuensis);* AF144520 *(Abies beshanzuensis);* AF144519 *(Abies beshanzuensis); AF144518(Abies holophylla);* AF144517 *(Abies holophylla);* AF144516 *(Abies firma);* AF144515 *(Abies firma);* AF144514 *(Abies firma);* AF144513 *(Larix gmelini);* AF144512 *(Larix gmelini);* AF144511 *(Pseudotsuga sinensis);* AF144510 *(Pseudotsuga sinensis);* AF144509 *(Pseudotsuga sinensis);* AF144508 *(Pseudotsuga menziesii);* AF144507 *(Pseudotsuga menziesii);* AF144506 *(Pseudotsuga menziesii);* AF144505 *(Cathaya argyrophylla);* AF144504 *(Picea smithiana);* AF144500 *(Pinus banksiana);* AF144499 *(Pinus banksiana);* AY224135 *(Pelargonium x hortorum);* AY764488 *(Pinus* taeda); AY764487 *(Pinus taeda);* AY764486 *(Pinus taeda);* AH014229 *(Pinus taeda);* AY764485 *(Pinus taeda);* AY764484 *(Pinus taeda);* AY764483 (*P*inus *taeda);* AH014228 *(Pinus taeda);* AY764482 *(Pinus taeda);* AY764481 *(Pinus* taeda); AY764480 *(Pinus taeda);* AY575768 *(Populus tomentosa);* D50034 *(Nicotiana tabacum);* D50033 *(Nicotiana tabacum);* EU280886 *(Larix sibirica);* EU280885 *(Larix sukaczewii);* EU280884 *(Larix sukaczewii);* EU280883 *(Larix sukaczewii);* EU280882 *(Larix sukaczewii);* EU280881 *(Larix sukaczewii);* EU280880 *(Larix sukaczewii);* EU280879 *(Larix sukaczewii);* EU280878 *(Larix sukaczewii);* EU280877 *(Larix sukaczewii);* EU280876 *(Larix sukaczewii);* EU280875 *(Larix sukaczewii);* EU280874 *(Larix sukaczewii);* EU280873 *(Larix sibirica);* EU280872 *(Larix sibirica);* EU280871 *(Larix sibirica);* EU280870 *(Larix sibirica);* EU280869 *(Larix sibirica);* EU280868 *(Larix sibirica);* EU280867 *(Larix sibirica);* EU280866 *(Larix kaempfen);* EU280865 *(Larix kaempfen);* EU280864 *(Larix gmelinii);* EU280863 *(Larix gmelinii);* EU280862 *(Larix gmelinii* var. Olgensis); EU280861 *(Larix gmelinii* var. Olgensis); EU280860 *(Larix gmelinii* var. Olgensis); EU280859 *(Larix gmelinii* var. Olgensis); EU280858 *(Larix gmelinii* var. Olgensis); EU280857 *(Larix gmelinii* var. Olgensis); EU280856 *(Larix kamtschatica);* EU280855 *(Larix kamtschatica);* EU280854 *(Larix kamtschatica);* EU280853 *(Larix gmelinii);* EU280852 *(Larix gmelinii)* var. japonica; EU280851 (La*r*ix *gmelinii*); EU280850 *(Larix gmelinii* var. Japonica); EU280849 *(Larix gmelinii);* EU280848 *(Larix* gmelinii); EU280847 *(Larix cajanderi);* EU280846 *(Larix cajanden);* EU280845 *(Larix cajanden);* EU280844 *(Larix cajanderi);* EU280843 *(Larix cajanden);* EU280842 *(Larix cajanden);* EU280841 *(Larix cajanden);* EU392783 *(Pinus elliottii);* EU392782 *(Pinus elliottii);* EU392781 *(Pinus sylvestris);* EU392780 *(Pinus sylvestris);* EU392779 *(Pinus radiata);* EU392778 *(Pinus radiata);* AY832332 *(Pseudotsuga menziesii* var. Menziesii); AY832331 *(Pseudotsuga menziesii* var. Menziesii); AY832330 *(Pseudotsuga menziesii* var. Menziesii); AY832329 *(Pseudotsuga menziesii* var. Menziesii); AY832328 *(Pseudotsuga menziesii* var. Menziesii); AY832327 *(Pseudotsuga menziesii* var. Menziesii); AY832326 *(Pseudotsuga menziesii* var. Menziesii); AY832325 *(Pseudotsuga menziesii* var. Menziesii); AY832324 *(Pseudotsuga menziesii* var. Menziesii); AY832323 *(Pseudotsuga menziesii* var. Menziesii); AY832322 *(Pseudotsuga menziesii* var. Menziesii); AY832321 *(Pseudotsuga menziesii* var. Menziesii); AY832320 *(Pseudotsuga menziesii* var. Menziesii); AY832319 *(Pseudotsuga menziesii* var. Menziesii); AY876936 *(Zingiber officinale);* U61383 *(Oryza rufipogon);* DQ983412 *(Pinus longaeva);* DQ983411 *(Pinus balfouriana);* DQ983410 *(Pinus* balfouriana); DQ983409 *(Pinus balfouriana);* DQ983408 *(Pinus balfouriana);* DQ983407 *(Pinus balfouriana);* DQ223433 *(Liriodendron tulipifera);* DQ667963 *(Vitis vinifera);* EF214739 *(Linum usitatissimum);* EF214738 *(Linum usitatissimum);* EF214737 *(Linum usitatissimum);* DQ840569 *(Hibiscus cannabinus);* DQ267975 *(Leucaena leucocephala).*

In the chimeric genes according to the current invention, the 4-CL coding region is operably linked to a heterologous plant-expressible promoter, wherein said promoter is a wound-inducible plant-expressible promoter, such as a wound-inducible and a xylem-selective plant-expressible promoter.

As used herein, the term "promoter" denotes any DNA which is recognized and bound (directly and indirectly) by a DNA-dependent RNA-polymerase during initiation of transcription. A promoter includes the transcription initiation site, and binding sites for transcription initiation factors and RNA polymerase, and can comprise various other sites (e.g. enhancers), at which gene expression regulatory proteins may bind.

The term "regulatory region", as used herein, means any DNA, that is involved in driving transcription and controlling (i.e. regulating) the timing and level of transcription of a given DNA sequence, such as a DNA coding for a protein or polypeptide. For example, a 5' regulatory region (or "promoter region") is a dNA sequence located upstream (I.e. 5') of a coding sequence and which comprises the promoter and the 5'-untranslated leader sequence. A 3' regulatory region is a DNA sequence located downstream (i.e. 3') of the coding sequence and comprises suitable transcription termination and or regulation signals, including one or more polyadenylation signals.

As used herein, the term "plant-expressible promoter" means a DNA sequence that is capable of controlling (initiating) transcription in a plant cell. This includes any promoter of plant origin, but also any promoter of non-plant origin which is capable of directing transcription in a plant cell, i.e. certain promoters of viral or bacterial origin such as the CaMV35S promoter (Hapster et al. 1988, Mol. Gen. Genet. 212, 182-190), the subterranean clover virus promoter No 4 or No 7 (WO9606932) or T-DNA gene promoters.

A "heterologous" promoter as used herein refers to a promoter other than the promoter which is naturally associated with the coding region for the 4-CL nucleotide sequence used in accordance with this invention.

A "xylem selective promoter" as used herein is a plant expressible promoter that is preferentially, but not necessarily exclusively driving transcription in xylem tissue of the plant. As an example of such a xylem selective promoter, the promoter region of the Populus tomentosa cinnamate-4-hydroxylase ("C4H") gene was used (see also SEQ ID NO. 3.) (Zhao et al., 2005 Plant Science 168, 1157-1162). Other xylem selective promoters include the promoter described in US patent application 20070180580, the promoters from the genes encoding xylem peptidase XCP1 and XCP2 as described in Funk et al. 2002, Plant Physiology 128(1) 84-94, the promoters described in US patent application 20080196125, the promoter of the glycin rich protein 1.8 from Sopho japonica as described by Lu et al. Plant Growth Regulation Volume 41 (3) pp 279-286.

As used herein a "wound-inducible promoter" is a promoter which drives a higher level of expression in plant tissue surrounding lesions than in intact plant tissues. The promoter region of the Populus tomentosa cinnamate-4-hydroxylase ("C4H") has been described to be wound-inducible (Zhao et al., 2005 Plant Science 168, 1157-1162). However, wound-inducible promoters are well defined in the art and also include the promoters form potato proteinase-inhibitor genes *(pin1* and *pin2)* and the like.

As used herein, "providing a plant cell with a chimeric gene" includes methods of introducing a chimeric gene transiently or stably into a plant cell using well established transformation methods.

To realize the above objects, the following technical measures are employed to obtain lignin monomer synthetic gene *4-CL* from *Populus tomentosa:*
1) Upon searching for the published poplar 4-CL gene sequence in NCBI database, Oligo6.0 software (commercially purchased, *sic passim)* is used to design primers at both sides of gene encoding sequence (forward primer: [5'-ATGAATCCACAAGAAGAATTCATC-3'] (SEQ ID NO. 4); and reverse primer: [5'-TTATATGCCTGCCAACTTTTCTTTCAG -3'] (SEQ ID NO. 5).
2) An RT-PCR amplification is performed using first-strand of cDNA as template, and the fragment resulted from the amplificationis sequenced to obtain *4-CL* gene sequence. The base sequence of the DNA molecule is shown in SEQ ID NO. 1.

Lignin monomer synthetic gene *4-CL* from *Populus tomentosa* is obtained by the above method. Transgenic *Brassica napus* with elevated *4-CL* gene expression is provided, characterized in that: the transgenic plant has its lignin content increased more than 10% compared to recipient control (non-transgenic plant); resistance thereof to *sclerotinia sclerotiorum* and lodging improved; lesion extension area thereof decreased around 30% compared to recipient control (non-transgenic plant); and stem strength thereof increased around 20% compared to control (non-transgenic plant).

In the present invention, the following technical solution is used for the above-mentioned purpose. A plasmid expression vector pBI121 (commercially purchased from Institute of Genetics and Developmental Biology, Chinese Academy of Sciences (CAS), ) is provided which contains specific expression promotor (C4H promoter, specifically expressed in xylem) and translation control elements.

Methods for introducing the DNA vector comprising the chimeric gene according to the invention into plant cells, particularly into Brassicaeae plant cells are well established in the art and include gene gun bombardment methods, and Agrobacteria-mediated introduction method. A recombinant DNA comprising a chimeric gene according to the invention can be stably incorporated in the nuclear genome of a cell of a plant, particularly a plant that is susceptible to Agrobacterium-mediated transformation. Gene transfer can be carried out with a vector that is a disarmed Ti-plasmid, comprising a chimeric gene of the invention, and harbored by *Agrobacterium.* This transformation can be carried out using the procedures described, for example, in EP 0,116,718. Ti-plasmid vector systems comprise a chimeric gene between the T-DNA border sequences, or at least to the left of the right T-DNA border. Alternatively, any other type of vector can be used to transform the plant cell, applying methods such as direct gene transfer (as described, for example, in EP 0,233,247), pollen-mediated transformation (as described, for example, in EP 0,270,356, WO85/01856 and US 4,684,611), plant RNA virus-mediated transformation (as described, for example, in EP 0,067,553 and US 4,407,956), liposome-mediated transformation (as described, for example, in US 4,536,475), and the like. Agrobacterium mediated introduction is preferred in the present invention, such as Agrobacterium LBA4404 mediated introduction into Brassica napus cotyledon petiole. A method for Agrobacterium mediated transformation of Brassica plants is described in De Block et al. 1989 Plant Physiol. 914: 694-701, entitled "Transformation of Brassica napus and Brassica oleracea Using Agrobacterium tumefaciens and the Expression of the bar and neo Genes in the Transgenic Plants"

Suitable for the invention is a method for preparing transgenic *Brassica napus* which can overexpress *Populus tomentosa 4-CL* gene, characterized in that a wound-inducible heterologous promoter is used, and the resistance to *Sclerotinia sclerotiorum* improves, which method comprises:
1) lignin monomer synthetic gene *4-CL* from *Populus tomentosa,* obtained from cloning, is ligated to plasmid pBI121, forming a vector capable of transforming or expressing under the control of C4H promoter, which is specifically expressed in xylem (pBI121-C4H: 4CL);
2) *Agrobacterium tumefaciens* LBA4404 is transformed with the vector prepared in step 1) (pBI121-C4H: 4CL), then used to co-infect a *Brassica napus* plant;
3) a positive plant comprising the chimeric gene is selected..

Suitable for the invention is the use of (1) *Populus tomentosa,* a unique Chinese tree species. Based on the published conserved sequence of poplar 4-CL gene, specific primers at conserved region flanking both sides of gene encoding sequence are designed. An RT-PCR amplification is performed, and the gene sequence obtained is shown in SEQ ID NO. 1. PCR product of C4H promoter and PBI121 plasmid are respectively digested by both restriction enzymes HindIII/BamHI. The digested fragment of PCR product and the longer fragment of digested PBI121 plasmid are ligated, and the resulting reaction product is used to transform competent cells DH5α (commercially obtained), and amplified. Then *4-CL* gene is introduced into vector PBI121 to replace the original GUS sequence in PBI121, while the 35S promoter sequence formerly in the vector is replaced by C4H promoter sequence. The constructed plant expression vector is renamed as pBI-C4H: 4-CL. (2) *Agrobacterium tumefaciens* LBA4404 is transformed with vector containing *4-CL* gene, and grown in YEP liquid medium (tryptone 10 g, yeast extract 10 g, sodium chloride 5 g); then aseptic seedling is soaked in *Agrobacterium* suspension. (3) Positive clones are screened in kanamycin (Kan) selective medium.

In a specific example, the aseptic seedling of the plant *used was Brassica napus. Brassica napus* plants with lignin monomer synthetic gene 4-CL expressed are selected in selective medium. The plants show lignin content increase of more than 10%, and their resistance to lodging and to *Sclerotinia sclerotiorum* increase around 20% and 30%, respectively.

The term "transgenic plant" used in this invention refers to those plants containing an inserted gene, wherein the expression of said gene can be stably enhanced or inhibited, so as to generate a specific biological trait.

The term "plant cell" used in this invention refers to all type of plant cells including but not limited to various immature embryo, callus or suspension cells (e.g., those of mature seed embryo, immature embryo, young ear, anther, coleoptile or young leaves), or protoplasts (mesophyll cell) of a plant. These plant cells can all regenerate to a plant under appropriate conditions.
Methods for cloning lignin monomer synthetic gene 4-CL from *Populus tomentosa* suitable for the present invention, are common methods used in the field. Methods for extracting mRNA include many well-founded techniques (such as using TRIzol Reagent, Invetrogen), and mRNA extraction kit can be, for example, commercially obtained. Construction of cDNA library is also a commonly-used molecular biology technique. Methods for constructing the vector described in the present invention and introducing the vector to plant are also commonly-used methods in the field. The plasmids involved (such as plasmid expression vector pBI121), and media for transformation (such as *Agrobacterium tumefaciens* LBA44040 and reagents used) are commercially available.

Disclosed is the use of a DNA comprising sequence of lignin monomer synthetic gene *4-CL* from *Populus tomentosa.* As lignin monomer synthetic gene *4-CL* is at the metabolic branching point in Phenylalanine pathway, encoding the key enzyme of lignin monomer synthesis pathway, and it's considered the ideal target gene for regulating lignin synthesis.. 4-CL is responsible for catalyzing various cinnamic acids and their derivatives to produce respective coenzyme A esters, involved in synthesis of downstream secondary product, and increases the total amount of lignin. Experimental results of the present invention shows that, lignin content of transgenic

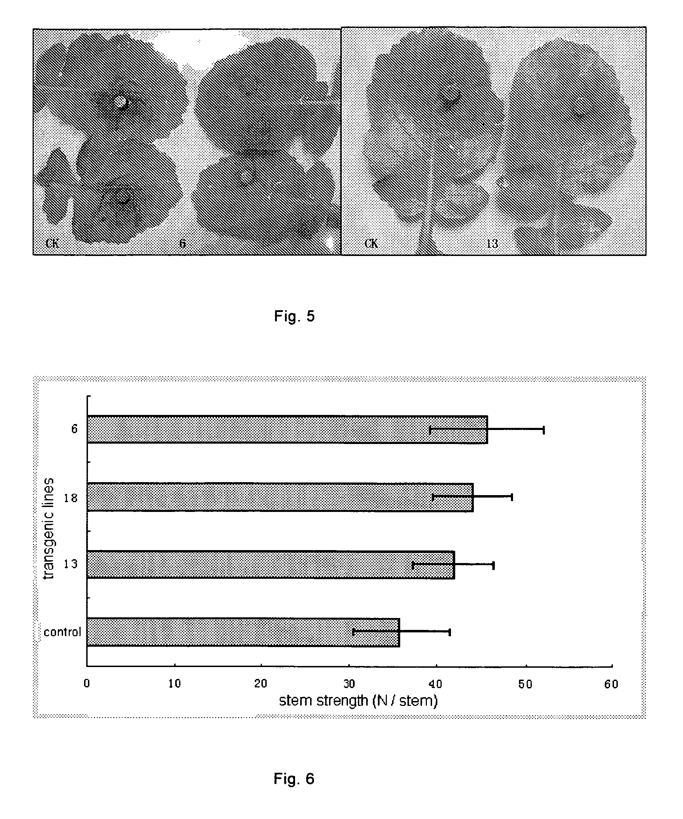

*Brassica napus* increases compared to recipient control (non-transgenic plant), *Brassica napus* line with highest increase shows an increase magnitude of 13.41% (see table 1); lesion extension area of transgenic plant decreases compared to recipient control (non-transgenic plant), the highest decrease magnitude is around 30% (see Fig.5); stem strength generally increases around 20% compared to control (non-transgenic plant) (see Fig.6), and it significantly improves the resistance to *Sclerotinia sclerotiorum* and lodging in *Brassica napus.* This method sheds new light on study of crop breeding and resistance to disease.

Preferred *Brassicaceae* to be treated in accordance with this invention, besides *Brassica napus,* include *Brassica juncea, Brassica oleraceae, Brassica carinata, Brassica nigra, Brassica campestris* and the like, and any intergenic crosses or synthetic varieties thereof. Other *Brassicaceae,* which may be treated according to the methods of the inventions include *Brassica cretica* (mustard), *Brassica elongata* (elongated mustard), *Brassica narinosa* (broadbeaked mustard), *Brassica nigra* (black mustard), *Brassica rapa* (field mustard), *Brassica rupstris* (mustard), Brassica *tournefortii* (Asian mustard). *Brassica napus* (2n=38, genome AACC) is an amphidiploid species, which originated from a spontaneous hybridization of *Brassica rapa* L. (syn. *B. campestris;* 2n=20, AA) and *Brassica oleracea* L. (2n=18, CC). B. napus contains the complete chromosome sets of these two diploid genomes.

As used herein, "a plant from the family *Brassicaceae"* or "a *Brassicaceae* plant" is a plant which according to the current botanical standard would be classified into the family *Brassicaceae* (formerly *Cruciferaeae). Brassicaceae* (Mustard) family members are easy to distinguish. They are annual or perannual plants with alternate leaves without stipules and posses simple inflorescence or branched racemes. The flowers are bilaterally symmetrical and hypogynous. With few exceptions, the flowers have 4 petals (free) alternating with 4 sepals (free) ; 6 stamens (4 long and 2 short), an ovary of 2 united carpels with parital placenta, 2 locular through the formation of a membranous false septum ; fruit is a dehiscent capsule opening by 2 valves. *Brassicaceae* include *inter alia* the following genera : *Sisymbrium, Descurania, Alliaria, Arabidopsis, Myagrum, Isatis, Bunia, Erysium, Hesperis, Malcolmia, Matthiola, Chorispora, Euclidium, Barbarea, Rorippa, Armoracia, Nasturtium, Dentaria, Cardamine, Cardaminopsis, Arabis, Lunaria, Alyssum, Berteroa, Lobularia, Draba, Erophila, Cochlearia, Camelina, Neslia, Capsella, Hornungia, Thlsapi, Iberis, Lepidium, Cardaria, Coronopus, Subularia, Conringia, Diplotaxis, Brassica, Sinapsis, Eruca, Erucastrum, Coincya, Hirschfeldia, Cakile, Rapistum, Crambe, Enarthrocarpus, Rhaphanus and Clausia.*

"Brassica plant" as used herein refers to a plant from the genus Brassica and includes the species *Brassica napus, Brassica oleracea, Brassica rapa, Brassica junceae and Brassica carinata.*

"Oilseed rape" as used herein, should be understood to include the species *Brassica napus, Brassica junceae* and *Brassica campestris.*

It is also an object of the invention to provide plant cells and plants containing the chimeric DNA according to the invention. Gametes, seeds (including crushed seeds and seed cakes), embryos, either zygotic or somatic, progeny or hybrids of plants comprising the chimeric genes of the present invention, which are produced by traditional breeding methods are also included within the scope of the present invention.

The plants obtained by the methods described herein may be further crossed by traditional breeding techniques with other plants to obtain fungal disease resistant progeny plants comprising the chimeric genes of the present invention.

The following non-limiting Examples describe chimeric genes comprising a coding region encoding a 4-CL enzyme, and the use thereof in Brassica plants. Unless otherwise specified, standard conditions are used as described in Sambrook et al., Molecular Cloning: A Laboratory Manual (New York: Cold Spring Harbor Laboratory Press, 1989), in Draper, J et al. (Blackwell Scientific Publications, 1988), Volumes 1 and 2 of Ausubel et al. (1994) Current Protocols in Molecular Biology, Current Protocols, USA, or as suggested by the manufacturer of the reagents and kits involved.. Standard materials and methods for plant molecular work are described in Plant Molecular Biology Labfax (1993) by R.D.D. Croy, jointly published by BIOS Scientific Publications Ltd (UK) and Blackwell Scientific Publications, UK.

Throughout the description and Examples, reference is made to the following sequences represented in the sequence listing:
SEQ ID NO. 1: nucleotide sequence of the 4-CL cDNA from *Populus tomentosa;*
SEQ ID NO. 2: amino acid sequence of the 4-CL protein from *Populus tomentosa;*
SEQ ID NO. 3: promoter region of the cinnamate-4-hydroxylase gene from *Populus tomentosa;*
SEQ ID NO. 4: forward primer for the PCR amplification of the 4-CL nucleotide sequence from *Populus tomentosa;*
SEQ ID NO. 5: reverse primer primer for the PCR amplification of the 4-CL nucleotide sequence from *Populus tomentosa;*
SEQ ID NO. 6: forward primer for detection of the chimeric C4H-4CL gene;
SEQ ID NO. 7: reverse primer for detection of the chimeric gene C4H-4CL.

### Examples

### Example 1: gene cloning and sequencing

Upon searching for the published poplar *4-CL* gene sequences in NCBI database, Oligo6.0 software (commercially available) was used to design primers at both sides of gene encoding sequence (forward primer: [5'-ATGAATCCACAAGAAGAATTCATC-3'] SEQ ID NO. 4; and reverse primer: [5'-TTATATGCCTGCCAACTTTTCTTTCAG -3'] SEQ ID NO. 5), which were then used to amplify the corresponding sequence of 4-CL from *Populus tomentosa.*
1. mRNA extraction from *Populus tomentosa.*
   RNA extraction (extract RNA by TRIZOL™ Kit)
   100 mg plant material was ground in liquid nitrogen.
   A. Add 1 ml TRIZOL, sit for 5 min at room temperature (20-25 °C,).
   B. Add 200 µl chloroform, agitate vigorously for 30 seconds, allow to remain at room temperature for 2 min.
   C. Centrifuge at 12,000 g, 15 min, 4 °C. Remove supernatant to a new tube, add 500 µl isopropanol, mix and allow to remain at room temperature for 15 min.
   D. Centrifuge at 12,000 g, 15 min, 4 °C. Discard supernatant, add 1 ml 70% alcohol.
   E. Centrifuge at 7,500 g, 7 min, 4 °C. Discard supernatant, air dry.
   F. Resuspend in DEPC-H₂O.
2. Use RevertAid H Minus First Strand cDNA Synthesis Kit (Fermentas) for reverse transcription of First Strand cDNA, following the manufacturer's instructions for operation.
3. Perform PCR amplification using cDNA as template, the obtained gene sequence is as shown in SEQ ID NO. 1.

### Example 2: vector construction

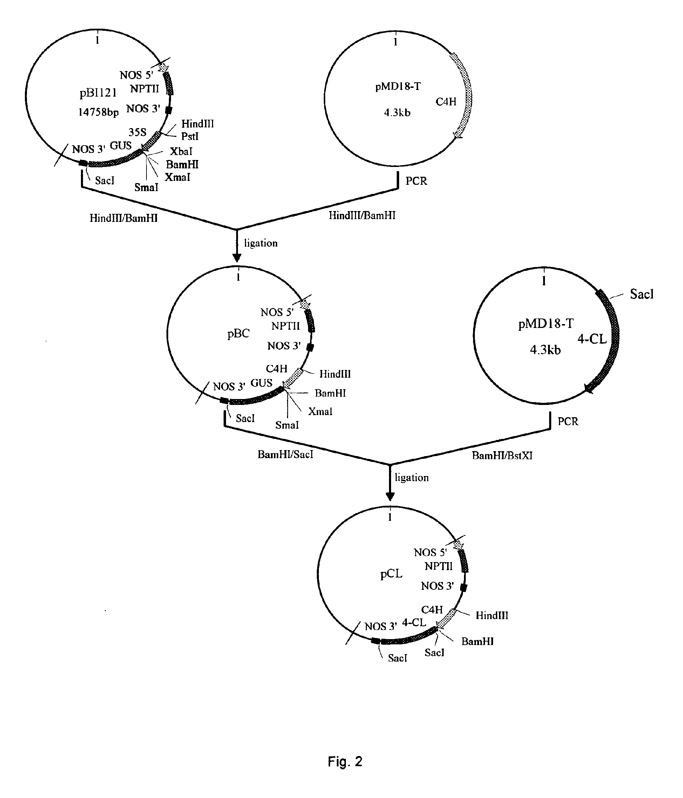

HindIII and BamHI restriction site sequences were introduced to 5' end and 3' end of C4H promoter (SEQ ID NO. 3) by PCR reaction. Then, the PCR amplification product of the C4H promoter and PBI121 plasmid were respectively digested by both restriction enzymes HindIII/BamHI. The digested fragment of PCR product and the digested longer fragment of PBI121 plasmid were ligated, and the resulting reaction product was used to transform competent cells E. coli DH5α (commercially obtained), and amplified. Then 4-CL gene was introduced into vector PBI121 to replace the original GUS sequence in PBI121, while the CaMV 35S promoter sequence formerly in the vector was replaced by C4H promoter sequence (as shown in Fig.2). The inserted fragment was assessed by DNA sequencing.

### Example 3: transformation and selection of the plant

### Agrobacterium LBA4404 transformation of Brassica napus

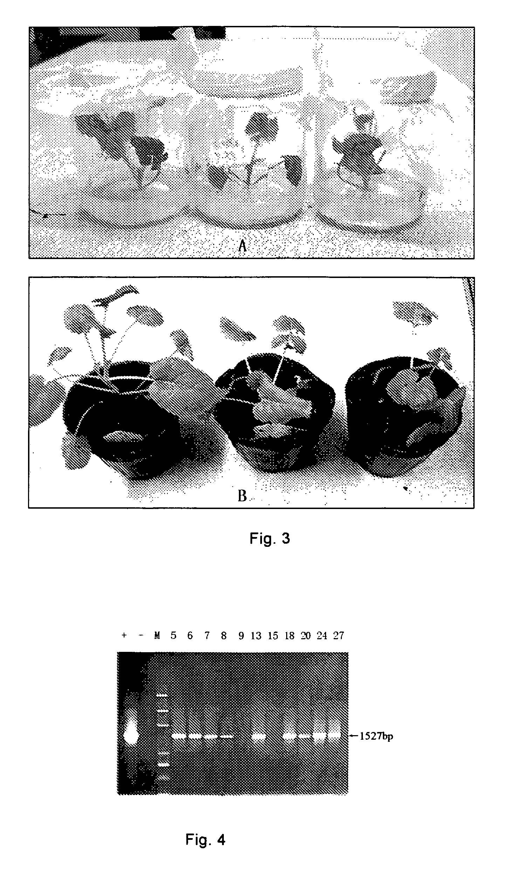

A. Preparation of aseptic seedlings: seed was soaked in 70% ethanol for 1 min, then in mercuric chloride (HgCl₂) for 13-15 min, rinsed 5 times with ddH₂O, plated in MS culture medium (PH 5.8), agar concentration of 0.8%. Aseptic *Brassica napus* seedling thus prepared was ready for use.
B. Transformation of *Brassica napus* cotyledon petiole: *Agrobacterium tumefaciens* LBA4404 (containing a T-DNA vector comprising the *4-CL* gene operably linked to the C4H promoter as described in Example 2) was inoculated on solid LB medium. After 2 days, a single colony was picked into 50 ml YEP liquid medium (tryptone 10 g + yeast extract 10 g + NaCl 5g + MgSO₄ 0.5 g) for culture. Cotyledon petioles were taken from aseptic seedling of 4-5 days, soaked in bacteria suspension for 5-8 min, with gently shaking in between. After that, the bacterial suspension was discarded and residual bacterial suspension on explant was removed with sterile filter paper. The resulting explant was placed on co-cultivation medium (MS + 0.2 mg/L 6-benzyladenine (6-BA) + 1 mg/L 2, 4-Dichlorophenoxyacetic acid (2, 4-D)+ 200 µm Acetosyringone (AS), PH5.8), and cultured for 2-3 days.
C. The co-cultivated explant was transferred to a different medium with carbenicillin (Car) only and without Kanamycin. Bacteria elimination and differential culture took place in dark greenhouse for 5-7 days. Then the co-cultivated explants were subjected to selection in selective medium with Kan selection pressure (MS + 3 mg/L 6-BA + 0.1 mg/L α-naphthaleneacetic acid (NAA)+5 mg/L silver nitrate (AgNO₃)+ 400 mg/L Car+15 mg/L Kan; pH 5.8), and wait for regenerated green buds to differentiate.
D. Regenerated buds were allowed to grow to a size of about 1 cm, cut off and transferred to root media (MS + 0.2 mg/L NAA + 10mg/L Kan+ 400 mg/L Car; pH 5.8), followed by further selection on solid MS culture medium supplemented with Car and Kan (see Fig.3A).
E. The rooted shoots were transferred outdoor, and then to soil containters after 10 days (see Fig.3B).

### Example 4: nucleic acid analysis - PCR assessment of the transformed plant.

### (1) Total DNA extraction was extracted from the transformed plant for PCR analysis according to the following protocol:

A. wipe the leaves with 70% alcohol, weigh out around100 mg
B. add 600 µl extraction buffer (0.2 M Tris-Cl, 0.25 M NaCl, 25 mM EDTA, 0.5% SDS, pH 7.5), grind quickly at room temperature.
C. mix by vortexing for 5-10s in 1.5 ml Ependorff tube.
D. Centrifuge at 12,000 rpm, 25min, room temperature. Take supernatant, add isopropanol of same volume, precipitate overnight at -20 °C.
E.Centrifuge at 12,000rpm, 15 min, room temperature. Add 200 µl 70% alcohol, wash DNA precipitate.
F. Centrifuge at 12,000rpm, 15 min, room temperature. Remove alcohol. Place tube upside down on paper towel, and wait for alcohol to evaporate.
G. Add 100 µl sterile water to resuspend DNA pellet. The concentration is determined by spectrophotometer or estimated by electrophoresis.
H. Perform PCR using total DNA as template.

### (2) PCR program

PCR reaction mixture was formulated the same as those for the PCR assessment of the plasmid (containing 4-CL gene). PCR primers were designed and synthesized based on the target gene and its upstream C4H promoter sequence of the plant expression vector: forward primer: [5'-CAGCTTCTTCTGCTTTCAATTACTCTC-3'] (SEQ ID NO. 6) and reverse primer [5'-CAACAGGAACTTCTCCCGCATT-3'] (SEQ ID NO. 7), time and temperature of the reaction were as follows:
94 °C 3 min,
94 °C 45 s,
59 °C 45 s,
72 °C 2 min 30 s, 30 cycles,
72 °C 5 min.
The test result shows that in positive control experiment and in samples from most transformed plant a DNA fragment of expected size could be amplified as shown by electrophoresis (pBI-*C4H: 4CL:* 1527 bp). , In negative control the DNA fragment could not be amplified, which means that the genome of transgenic *Brassica napus* contained DNA fragment of said exogenous chimeric gene. The result is shown in Fig. 4.

### Example 5: Assessment of lignin content, Sclerotinia sclerotiorum resistance and stem strength of T0 transgenic line.

### (1) Measurement of lignin content

Lignin conted was measured according to the following protocol: A Stem part was cut off, and dried in oven till it reached constant weight, pulverized thoroughly by pulverizer; then 0.3 g of the sample was weighed out, to which 7.5 ml 72% sulphuric acid was added, and digested for 1 h at 30 °C. The digestion solution was diluted with distilled water till the concentration of sulphuric acid is 4%, following treatment for 1 h at 121 °C, the sample was cooled to room temperature, filtered with one layer of filter paper; the residue was dried and weighed, and lignin content was calculated. Among 31 (PCR positive) 37301 T0 transgenic lines obtained, some of the transgenic lines were randomly selected for lignin content measurement (see table 1) in this experiment. As can be seen from the table, the lignin contents of transgenic lines 6, 13, and 18 were all increased compared to control, whereby lines 6 and 13 had the highest increase magnitude, which increase amount to 13.41% and 11.48% based on the lignin content of the control, respectively.

**Table1 lignin content analysis of transgenic Brassica napus**

| | CK | 6 | 13 | 18 |
|---|---|---|---|---|
| Lignin content (%) | 9.32±0.69 | 10.57±0.52 | 10.39±0.72 | 10.35±0.59 |

### (2) Assessment of Sclerotinia sclerotiorum resistance

The Sclerotinia resistance test was conducted by inoculating *in vitro a Brassica napus* leaf with *Sclerotinia sclerotiorum* at the seedling stage. Before bolting of the *Brassica napus,* leaves were excised from the same spot, put on white porcelain plates, with two sets on each plate, and 8 leaves each set, petioles were wrapped with wet gauze to stay moisturized. Sclerotinia mycelia pieces were invertedly deposited on mesophyll at upper part of the main vein of the leaves. After inoculation, leaves were covered with transparent plastic film to stay 100% moisturized. After incubation for 2 days at 23 °C, the diameter (cm) of the lesions was measured . Measurement was repeated three times, each time repeated with 5-7 leaves, and average area of their lesions was calculated. The result shows that, compared with non-transgenic plant as control, transgenic lines 6, 13, 18 have lesions with decreased extension area (P 〈a=0.05〉 , which are respectively 66.42%, 77.87%, 72.08% of the control (see Fig.5).

### (3) Test for stem strength

During the maturation period of *Brassica napus,* the stem strength of *Brassica napus* was determined by lodging tester DIK-7401 (purchased from Japan). Normally growing *Brassica napus* was selected, with 15 plants in each region. The upper canopy part at the middle part of stem was cut off, the plant was bend to a certain angle (45°) by lodging tester to measure stem strength (N/stem), and calculate the average value. The result shows that, the stem strength increases compared to control (P〈α=0.05〉 , and the increase magnitude are 27.58%, 17.16%, 22.84%, respectively (see Fig.6).

## Claims

1. A method to increase resistance against fungal disease caused by Sclerotinia sclerotiorum in a Brassica plant comprising the step of providing cells of said Brassica plant with a chimeric gene by introducing said gene transiently or stably into a plant cell using transformation method, said chimeric gene comprising a nucleotide sequence encoding a 4-coumarate-CoA ligase, preferably a 4-coumarate-CoA ligase from plant origin, operably linked to a heterologous plant-expressible promoter and a transcription termination and polyadenylation region, wherein said plant-expressible promoter is a wound-inducible plant-expressible promoter.

2. The method of claim 1, wherein said plant-expressible promoter is a wound-inducible plant-expressible promoter and a xylem selective plant-expressible promoter.

3. The method of claim 1, wherein said plant-expressible promoter comprises the nucleotide sequence of SEQ ID NO. 3.

4. The method of any one of claims 1 to 3, wherein said 4-coumarate-CoA ligase comprises the amino acid sequence of SEQ ID NO. 2.

5. The method of any one of claims 1 to 4, wherein said nucleotide sequence encoding a 4-coumarate-CoA ligase comprises the nucleotide sequence of SEQ ID NO. 1 from the nucleotide at position 11 to the nucleotide at position 1621, or comprises the sequence of SEQ ID NO. 1.

6. The method of any one of claims 1 to 5, wherein said Brassica plant is selected from *Brassica napus, Brassica junceae, Brassica oleraceae, Brassica rapa, Brassica nigra* or *Brassica carinata.*

7. A chimeric DNA comprising a nucleotide sequence encoding a 4-coumarate-CoA ligase, preferably a 4-coumarate-CoA ligase from plant origin, operably linked to a heterologous plant-expressible promoter and a transcription termination and polyadenylation region wherein said promoter is a wound-inducible plant-expressible promoter.

8. The chimeric DNA of claim 7, wherein said promoter is a wound-inducible plant-expressible promoter and a xylem-selective plant-expressible promoter.

9. The chimeric DNA of claim 7, wherein said promoter comprises the nucleotide sequence of SEQ ID NO. 3.

10. The chimeric DNA of any one of claims 7 to 9, wherein said 4-coumarate-CoA ligase comprises the amino acid sequence according to SEQ ID NO. 2.

11. A Brassicaceae plant cell, plant or seed thereof, preferably a Brassica plant cell, plant or seed thereof comprising a chimeric DNA according to any one of claims 7 to 10.

12. Use of the chimeric DNA according to any one of claims 7 to 10 to obtain resistance in *Brassica plants, preferably Brassica napus,* against *Sclerotinia sclerotiorum.*

## Patentansprüche

1. Verfahren zum Erhöhen der Resistenz gegen durch Sclerotinia sclerotiorum verursachter Pilzkrankheit in einer Brassica-Pflanze, umfassend den Schritt, dass man Zellen der Brassica-Pflanze mit einem chimären Gen dadurch bereitstellt, dass man das Gen unter Verwendung von Transformationsverfahren transient oder stabil in eine Pflanzenzelle einführt, wobei das chimäre Gen eine Nukleotidsequenz umfasst, die für eine 4-Cumarat-CoA-Ligase, vorzugsweise für eine 4-Cumarat-CoA-Ligase pflanzlichen Ursprungs codiert, die operativ mit einem heterologen in Pflanzen exprimierbaren Promoter und einer Transkriptionsterminations- und Polyadenylierungsregion verknüpft ist, wobei es sich bei dem in Pflanzen exprimierbaren Promoter um einen wundinduzierbaren in Pflanzen exprimierbaren Promoter handelt.

2. Verfahren nach Anspruch 1, wobei es sich bei dem in Pflanzen exprimierbaren Promoter um einen wundinduzierbaren in Pflanzen exprimierbaren Promoter und einen xylemselektiven in Pflanzen exprimierbaren Promoter handelt.

3. Verfahren nach Anspruch 1, wobei der in Pflanzen exprimierbare Promoter die Nukleotidsequenz von SEQ ID NO: 3 umfasst.

4. Verfahren nach einem der Ansprüche 1 bis 3, wobei die 4-Cumarat-CoA-Ligase die Aminosäuresequenz von SEQ ID NO: 2 umfasst.

5. Verfahren nach einem der Ansprüche 1 bis 4, wobei die Nukleotidsequenz, die für eine 4-Cumarat-CoA-Ligase codiert, die Nukleotidsequenz von SEQ ID NO: 1 von dem Nukleotid in Position 11 bis zu dem Nukleotid in Position 1621 umfasst oder die Sequenz von SEQ ID NO: 1 umfasst.

6. Verfahren nach einem der Ansprüche 1 bis 5, wobei die Brassicapflanze aus der Reihe *Brassica napus, Brassica junceae, Brassica oleraceae, Brassica* rapa, *Brassica nigra* oder *Brassica carinata* ausgewählt ist.

7. Chimäre DNA, die eine Nukleotidsequenz umfasst, die für eine 4-Cumarat-CoA-Ligase, vorzugsweise für eine 4-Cumarat-CoA-Ligase pflanzlichen Ursprungs codiert, die operativ mit einem heterologen in Pflanzen exprimierbaren Promoter und einer Transkriptionsterminations- und Polyadenylierungsregion verknüpft ist, wobei es sich bei dem Promoter um einen wundinduzierbaren in Pflanzen exprimierbaren Promoter handelt.

8. Chimäre DNA nach Anspruch 7, wobei es sich bei dem Promoter um einen wundinduzierbaren in Pflanzen exprimierbaren Promoter und einen xylemselektiven in Pflanzen exprimierbaren Promoter handelt.

9. Chimäre DNA nach Anspruch 7, wobei der Promoter die Nukleotidsequenz von SEQ ID NO: 3 umfasst.

10. Chimäre DNA nach einem der Ansprüche 7 bis 9, wobei die 4-Cumarat-CoA-Ligase die Aminosäuresequenz von SEQ ID NO: 2 umfasst.

11. Brassicaceae-Pflanzenzelle, -pflanze oder Samen davon vorzugsweise eine Brassica-Pflanzenzelle, -pflanze oder Samen davon, die/der eine chimäre DNA nach einem der Ansprüche 7 bis 10 umfasst.

12. Verwendung der chimären DNA nach einem der Ansprüche 7 bis 10 zum Erzielen von Resistenz in Brassica-Pflanzen, vorzugsweise *Brassica napus,* gegen *Sclerotinia sclerotiorum.*

## Revendications

1. Procédé pour augmenter la résistance à une maladie fongique provoquée par Sclerotinia sclerotiorum dans une plante Brassica, comprenant l'étape de mise à disposition, à des cellules de ladite plante Brassica, d'un gène chimère, par introduction dudit gène d'une manière transitoire ou stable dans une cellule végétale par utilisation de méthodes de transformation, ledit gène chimère comprenant une séquence nucléotidique codant pour une 4-coumarate-CoA ligase, de préférence pour une 4-coumarate-Coa ligase d'origine végétale, liée de manière opérationnelle à un promoteur hétérologue exprimable dans les plantes et à une région de terminaison de transcription et de polyadénylation, ledit promoteur exprimable dans les plantes étant un promoteur exprimable dans les plantes et inductible par une blessure.

2. Procédé de la revendication 1, dans lequel ledit promoteur exprimable dans les plantes est un promoteur exprimable dans les plantes et inductible par une blessure, et un promoteur exprimable dans les plantes et xylème-sélectif.

3. Procédé de la revendication 1, dans lequel ledit promoteur exprimable dans les plantes comprend la séquence nucléotidique de SEQ ID NO:3.

4. Procédé de l'une quelconque des revendications 1 à 3, dans lequel ladite 4-coumarate-CoA ligase comprend la séquence d'acides aminés de SEQ ID NO:2.

5. Procédé de l'une quelconque des revendications 1 à 4, dans lequel ladite séquence nucléotidique codant pour une 4-coumarate-CoA ligase comprend la séquence nucléotidique de SEQ ID NO:1 du nucléotide en position 11 au nucléotide en position 1621, ou comprend la séquence de SEQ ID NO:1.

6. Procédé de l'une quelconque des revendications 1 à 5, dans lequel ladite plante Brassica est choisie parmi *Brassica napus, Brassica junceae, Brassica oleraceae, Brassica* rapa, *Brassica nigra* ou *Brassica carinata.*

7. ADN chimère comprenant une séquence nucléotidique codant pour une 4-coumarate-CoA ligase, de préférence pour une 4-coumarate-CoA ligase d'origine végétale, liée de manière opérationnelle à un promoteur hétérologue exprimable dans les plantes et à une région de terminaison de transcription et de polyadénylation, dans lequel ledit promoteur est un promoteur exprimable dans les plantes et inductible par une blessure.

8. ADN chimère de la revendication 7, dans lequel ledit promoteur est un promoteur exprimable dans les plantes et inductible par une blessure, et un promoteur exprimable dans les plantes xylème-sélectif.

9. ADN chimère de la revendication 7, dans lequel ledit promoteur comprend la séquence nucléotidique de SEQ ID NO:3.

10. ADN chimère de l'une quelconque des revendications 7 à 9, dans lequel ladite 4-coumarate-CoA ligase comprend la séquence d'acides aminés selon SEQ ID NO:2.

11. Cellule végétale de Brassicaceae, plante ou graine de cette dernière, de préférence cellule végétale de Brassica, plante ou graine de cette dernière, comprenant un ADN chimère selon l'une quelconque des revendications 7 à 10.

12. Utilisation de l'ADN chimère selon l'une quelconque des revendications 7 à 10 pour obtenir une résistance dans des plantes Brassica, de préférence *Brassica napus,* à *Sclerotinia sclerotiorum.*
